# EUROPEAN PATENT SPECIFICATION

(11) **EP 0 959 864 B1**
(45) Date of publication and mention of the grant of the patent: **22.01.2003**
(21) Application number: 97921064.8
(22) Date of filing: 28.04.1997
(51) Int. Cl.: A61G 13/00, A01N 1/02

(54) **PREPARATION TABLE**
VORBEREITUNGSTISCH
TABLE DE PREPARATION

(30) Priority: 25.06.1996 SE 9602510
(43) Date of publication of application: 01.12.1999
(73) Proprietor: Vivar, Roberto, 143 03 Värby (SE)
(72) Inventor: Vivar, Roberto, 143 03 Värby (SE)
(74) Representative: Sundström, Per Olof
(86) International application number: SE9700712
(87) International publication number: WO97049370

(56) References cited:
- US-A- 3 411 317

## Description

The present invention relates to a table which is adapted for the preparation of human tissue or organs intended for transplantation and which is of the kind defined in the preamble of Claim 1.

A table of the kind defined in the preamble of Claim 1 is known from UA-A 3,411,317. The table top includes an aperture that accommodates a basin into which the transplantation tissue or organ is placed. The outer surface of the basin is cooled by a circulating coolant, such as ethylene glycol. The circulating coolant cools a conventional cooling machine. The basin cannot be removed readily from the known table and used to transport the transplantation tissue or organ.

It is normally necessary to store the transplantation tissue or organ in a sterile saline water bath. According to conventional techniques, sterile ice is used in the basin to initially cool the bath and the transplantation tissue or organ, and then to keep the tissue or organ at a low temperature.

One problem encountered when preparing transplantation tissue or organs resides in establishing a sterile working environment at the table, so as to prevent contamination of the content of the basin during the preparatory work.

The object of the present invention is to provide a preparation table that will alleviate or eliminate this problem.

This object is achieved with a preparation table according to the accompanying Claim 1.

Further embodiments of the preparation table are set forth in the dependent Claims.

The invention and further embodiments thereof also provide other advantages, as made apparent below or as will be obvious to the skilled person.

The invention is based on a table for preparing transplantation tissue or organs of the kind that includes a basin which is placed in an aperture or opening in the tabletop, and cooling devices that function to cool the content of the basin through the basin wall.

With a starting point from a preparation table of this kind, the invention is characterized mainly in that the cooling devices have fixed cooling surfaces that lie in thermally conductive contact with the basin when said basin is positioned in the table opening; and in that the basin is removably recessed in the table opening in heat-exchange contact with the cooling surfaces of said cooling device; and in that a sterile cloth is placed on the tabletop, wherewith the basin rests on the cloth when in heat-exchange contact with the cooling surface of said cooling device.

The cloth, or fabric, may consist of a thin, impervious and flexible sterilized plastic foil, for instance polyethylene foil. The foil may simply be spread out over the table and the basin then placed on the foil, so that the foil will be entrained by the basin as it is lowered or recessed into the table opening and shape itself to the shape of the outside of the basin. Because the foil is thin, it will have a low heat insulation factor. The foil/cloth may be of a disposable kind.

In one embodiment of the invention, the cloth includes a part whose shape corresponds to the shape of the basin, so as to enable the basin to be placed in the basin-shaped part of the cloth, wherewith an exchange of heat takes place between the cooling surfaces of the cooling device and the wall of said basin via the basin-shaped part of the sterile cloth. In one alternative embodiment, the cloth may include an opening whose defining edge seals tightly against an outwardly facing, peripherally extending outer wall-part of the basin. Because the transfer of heat between the basin and the cooling device takes place by thermal conduction through solid material, the basin can be both easily removed from the table and easily placed in the table opening.

In one embodiment of the invention, the cloth, and particularly that part of the cloth which is placed between the basin and the cooling surfaces, is comprised of a material that has good thermal conductivity.

By giving the cooling unit/cooling device of the table a relatively high cooling capacity, the sterile aqueous solution can be cooled to a pre-selected temperature, preferably a temperature of about +4°C, and the sterile aqueous solution and the transplantation tissue or organ kept at said temperature under sterile conditions with respect to the table, cloth and basin.

The price of sterile-packed sterile ice is at present relatively high. The preparation of a liver intended for transplantion normally requires three packages of ice, each costing about SEK 600.

A further embodiment of the inventive table includes a supportive structure that is intended to be placed in the basin in a manner to hold the tissue or organ at an appropriate level in the basin in relation to the existing level of liquid therein. In preparing the transplantation tissue/ liver, it is normally necessary to keep the upper surface of the tissue/liver immediately beneath the surface of the liquid in the basin, at the same time as it must be possible to reach the upwardly facing liquid surface in order to enable the preparation work to be carried out The supportive structure may have the form of a set of supportive stands of mutually different effective heights. Alternatively, the support structure may comprise a device whose height can be adjusted.

The invention will now be described in more detail with reference to an exemplifying embodiment thereof and also with reference to the accompanying drawing, in which
Fig. 1 is a schematic, vertical sectional view of an inventive table and associated basin; and
Fig. 2 is a view taken on the line II-II in Fig. 1.

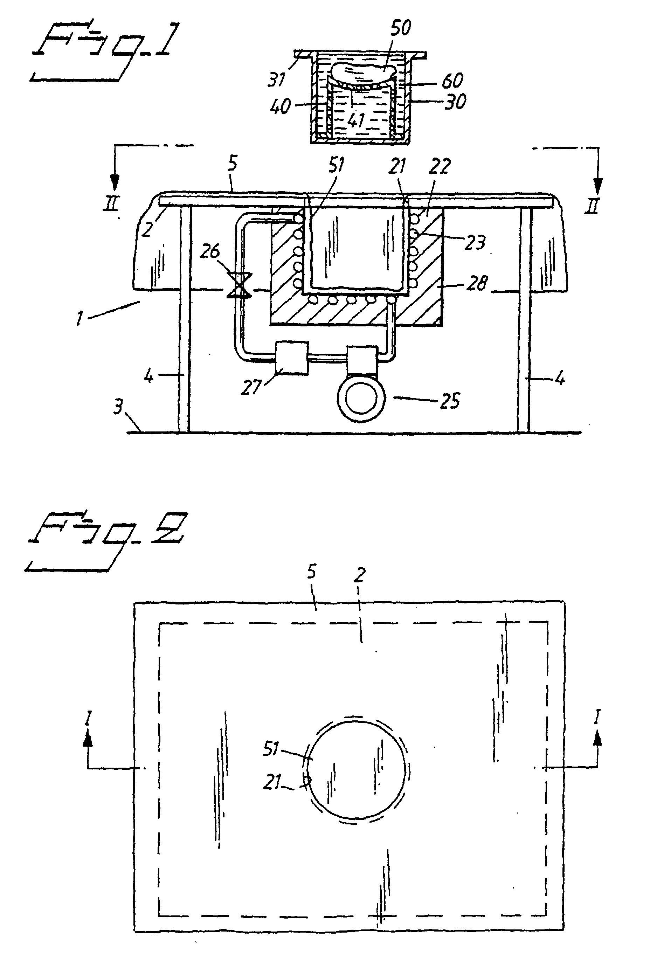

Fig. 1 is a vertical section view of a table 1 that includes a tabletop 2 which is supported at a convenient working height from a floor 3 by means of table legs 4. The tabletop includes an opening 21 whose defining edge adjoins the upper edge of a basin-shaped cooling element 22. Disposed on the outside of the cooling element 22 is a pipe coil 23 which is through-passed by a coolant which cools a cooling machine 25, e.g. a compressor, and which may be controlled and regulated in a known manner. A sterile cloth 5 is placed over the upper surface of the table 2. The cloth 5 may include a part 51 whose shape corresponds to the shape of the cooling element 22, such that said cloth part 51 will pass into the cooling element and conform to the upwardly facing surface thereof. The cloth 5, or the cloth part 51, may be comprised of a material that has a relatively good thermal conductivity. The cloth 5 will preferably consist of a thin, impervious, flexible plastic foil, for instance polyethylene foil. A cloth of this nature is able to readily shape itself to the shape of the basin, therewith enabling the cloth to be simply spread out on the table and the basin then inserted into the opening while entraining the cloth therewith, the remainder of the cloth draping around the outside of the basin 30. The cloth will, of course, have a size sufficient to enable the cloth to fully cover the table surface, even after the basin has been placed on the cloth and into the opening 21.

A basin 30 having an outer/bottom shape that corresponds to the shape of the cooling element 22 can be placed on the cloth 5 in the opening 21 in surface abutment with the cooling element 22 over substantially the whole of its area, through the medium of the part 51 of the cloth 5. The flange 31 on the basin may rest on the tabletop 2 via the cloth 5 in this regard. Also shown in Fig. 1 is heat insulation placed on the outside of the pipe coil 23, a condensor 27, and an expansion valve 26 for carrying heat away from the compressed refrigerant and vaporizing the refrigerant respectively.

The basin 30 has a round or circular-cylindrical shape, including an upper edge flange that rests on the table around said opening.

As shown in Fig. 1, a supportive structure 40 may be placed in the basin 30 so as to enable the transplantation tissue or organ 50 to be positioned at a desired level in the liquid mass 60 and so that the tissue or organ 50 will lie beneath the surface of the liquid and close to said surface, so that the tissue or organ can be reached easily for carrying out preparatory transplantation work or some like surgical procedure. The tissue 50 is supported on the upper surface 41 of the supportive structure 40, said upper surface preferably being slightly concave.

The supportive structure 40 may be made of sheet metal and will preferably include a plurality of through-penetrating openings that permit liquid to pass through the structure.

The supportive structure may also include a flange whose outer foot is guided by the basin wall. The foot flange carries a cylindrical wall, which, in turn, supports an upper wall whose upper surface is slightly concave. A set of such supports of mutually different heights can be provided so as to enable the user to chose a support whose foot will provide a convenient height.

The capacity of the cooling machine 25 will be sufficiently great to enable requisite cooling of the liquid mass 60 and the tissue 50 to be achieved in a basin 30 in the table 1 with the cloth 5 in a short period of time required to complete preparatory work on the tissue or organ 50 in the basin. The cooling machine will preferably have control means that enable the liquid mass to be maintained at a given temperature, beneficially a temperature of about +4°C.

## Claims

1. A table for preparing human tissue or organs (50) for transplantation or like surgical procedure, said table including a tabletop (2), a basin (30) which is placed into an opening (21) in the tabletop, and basin cooling devices (22, 23, 25-28), the cooling devices having fixed cooling surfaces (22, 23) that lie in heat conducting contact with the basin (30); **characterized in that** the basin is loosely placed in the table opening (21) and in contact with the cooling surfaces (22 and 23); and **in that** the tabletop (2) has arranged thereon a sterile cloth (5) on which the basin (30) rests.

2. A table according to Claim 1, **characterized in that** the cloth (5) is placed between the basin (30) and the cooling surfaces.

3. A table according to any one of Claims 1-2, **characterized in that** the basin (30) includes a supportive structure (40, 41) which enables the transplantation tissue or organ to be placed in the basin at a chosen height from the bottom of said basin (30.

4. A table according to any one of Claims 1-3, **characterized in that** the cooling devices on said table are dimensioned to produce sterile ice in the basin (30) in contact with the table cooling surfaces (22, 23) via the cloth (5).

5. A table according to any one of Claims 1-4, **characterized in that** the cloth (5) consists of a thin, impervious plastic foil.

6. A table according to Claim 5, **characterized in that** the foil is a sterilized polyethylene foil.

## Patentansprüche

1. Tisch zur Vorbereitung von menschlichem Gewebe oder Organen (50) zur Transplantation oder ähnlichen chirurgischen Verfahren, wobei der Tisch eine Tischplatte (2), ein Becken (30), welches in eine Öffnung (21) in der Tischplatte eingesetzt ist, und Becken-Kühlvorrichtungen (22, 23, 25-28) enthält, wobei die Kühlvorrichtungen feststehende Kühlflächen (22, 23) haben, welche in wärmeleitendem Kontakt mit dem Becken (30) liegen;
**dadurch gekennzeichnet, dass** das Becken in der Tischöffnung (21) lose platziert und in Kontakt mit den Kühlflächen (22 und 23) ist; und dass die Tischplatte (2) einen sterilen Stoff (5) darauf angeordnet hat, auf welchem das Becken (30) ruht.

2. Tisch nach Anspruch 1, **dadurch gekennzeichnet, dass** der Stoff (5) zwischen dem Becken (30) und den Kühlflächen platziert ist.

3. Tisch nach einem der Ansprüche 1 bis 2, **dadurch gekennzeichnet, dass** das Becken (30) eine Trägerstruktur (40, 41) enthält, welches es ermöglicht, dass das Transplantationsgewebe oder Organ im Becken auf einer gewählten Höhe vom Boden des Beckens (30) platziert wird.

4. Tisch nach einem der Ansprüche 1 bis 3, **dadurch gekennzeichnet, dass** die Kühlvorrichtungen an dem Tisch so dimensioniert sind, dass sie steriles Eis in dem Becken (30) in Kontakt mit den Tisch-Kühloberflächen (22, 23) über den Stoff (5) erzeugen.

5. Tisch nach einem der Ansprüche 1 bis 4, **dadurch gekennzeichnet ,dass** der Stoff (5) aus einer dünnen, undurchlässigen Kunststofffolie besteht.

6. Tisch nach Anspruch 5, **dadurch gekennzeichnet, dass** die Folie eine sterilisierte Polyethylenfolie ist.

## Revendications

1. Une table pour la préparation de tissu ou d'organes humains (50) en vue d'une transplantation ou d'un processus chirurgical analogue, ladite table comprenant un dessus de table (2), un récipient (30) qui est placé dans une ouverture (21) ménagée dans le dessus de table, et des dispositifs de refroidissement (22, 23, 25-28) du récipient, les dispositifs de refroidissement présentant des surfaces de refroidissement fixes (22, 23) qui se trouvent en contact de conduction thermique avec le récipient (30) ; **caractérisée en ce que** le récipient est placé de manière lâche dans l'ouverture (21) de la table et en contact avec les surfaces de refroidissement (22 et 23) ; et **en ce qu'**est disposée sur le dessus de table (2) une nappe stérile (5) sur laquelle repose le récipient (30).

2. Une table selon la revendication 1, **caractérisée en ce que** la nappe (5) est placée entre le récipient (30) et les surfaces de refroidissement.

3. Une table selon l'une quelconque des revendications 1-2, **caractérisée en ce que** le récipient (30) incorpore une structure de support (40, 41) qui permet au tissu ou aux organes de transplantation d'être placés dans le récipient à une hauteur choisie à partir du fond dudit récipient (30) .

4. Une table selon l'une quelconque des revendications 1-3, **caractérisée en ce que** les dispositifs de refroidissement prévus sur ladite table sont dimensionnés pour produire de la glace stérile dans le récipient (30) en contact avec les surfaces de refroidissement (22, 23) de la table par l'intermédiaire de la nappe (5).

5. Une table selon l'une quelconque des revendications 1-4, **caractérisée en ce que** le nappe (5) est constituée par une feuille mince en matière plastique imperméable.

6. Une table selon la revendication 5, **caractérisée en ce que** la feuille mince est une feuille mince de polyéthylène stérilisée.
